# EUROPEAN PATENT APPLICATION

(11) **EP 0 939 136 A1**
(43) Date of publication of application: **01.09.1999**
(21) Application number: 98200022.6
(22) Date of filing: 07.01.1998
(51) Int. Cl.: C12Q 1/68

(54) **Assay for predicting the angiographic response to lipid-lowering therapy in patients**

(71) Applicant: Academisch Ziekenhuis bij de Universiteit van Amsterdam, 1105 AZ Amsterdam ZO (NL)
(72) Inventor: Kuivenhoven, Jan Albert, Brookline, MA 02146 (US); Kastelein, Johannes Jacobus Pieter, Vancouver, BC V6R 1H7 (CA)
(74) Representative: Van Someren, Petronella F. H. M.

(57) **Abstract**

The present invention relates to an assay for testing the genetic predisposition to respond to lipid-lowering therapy in patients with coronary artery disease (CAD), comprising identifying the presence or absence of a TaqIB restriction site in intron 1 in both alleles of the cholesteryl ester transfer protein (CETP) gene by suitable molecular biological techniques; and correlating the presence of the restriction site on one or both alleles with a high or intermediate, susceptibility for lipid-lowering therapy. The invention further relates to a testkit for performing the assay and to a cholesterol ester transfer protein (CETP) gene marker for identifying individuals that may or may not be susceptible for lipid-lowering therapies.

SEQUENCE LISTING

(1) GENERAL INFORMATION:

(i) APPLICANT:
(A) NAME: Academisch Ziekenhuis bij de UvA - Academisch
Medisch Centrum
(B) STREET: Meibergdreef 15
(C) CITY: AMSTERDAM ZO
(E) COUNTRY: The Netherlands
(F) POSTAL CODE (ZIP): 1105 AZ

(ii) TITLE OF INVENTION: Assay for predicting the angiographic
response to lipid-lowering therapy in patients

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98200022.6

(2) INFORMATION FOR SEQ ID NO: 1:

(i) SEQUENCE CHARACTERISTICS:
(A) LENGTH: 1656 base pairs
(B) TYPE: nucleic acid
(C) STRANDEDNESS: unknown
(D) TOPOLOGY: linear

(ii) MOLECULE TYPE: DNA (genomic)

(vi) ORIGINAL SOURCE:
(A) ORGANISM: Homo sapiens

(ix) FEATURE:
(A) NAME/KEY: TATA_signal
(B) LOCATION:330..335

(ix) FEATURE:
(A) NAME/KEY: prim_transcript
(B) LOCATION:361..>1656
(D) OTHER INFORMATION:/note= "CETP mRNA and introns"

(ix) FEATURE:
(A) NAME/KEY: exon
(B) LOCATION:<388..505
(D) OTHER INFORMATION:/gene= "CETP"
/number= 1
/note= "cholesteryl ester transferase protein precursor;
GOO-119-773"

(ix) FEATURE:
(A) NAME/KEY: sig_peptide
(B) LOCATION:388..438
(D) OTHER INFORMATION:/codon_start= 1
/note= "cholesteryl ester transferase protein signal
peptide"

(ix) FEATURE:
(A) NAME/KEY: mat_peptide
(B) LOCATION:439..505
(D) OTHER INFORMATION:/codon_start= 1
/note= "cholesteryl ester transferase protein"

(ix) FEATURE:
(A) NAME/KEY: intron
(B) LOCATION:506..1407
(D) OTHER INFORMATION:/note= "CETP intron A"

(ix) FEATURE:
(A) NAME/KEY: exon
(B) LOCATION:1408..1522

(ix) FEATURE:
(A) NAME/KEY: mat_peptide
(B) LOCATION:1408..1522
(D) OTHER INFORMATION:/codon_start= 1
/note= "cholesteryl ester transferase protein"

(ix) FEATURE:
(A) NAME/KEY: intron
(B) LOCATION:1523..>1656
(D) OTHER INFORMATION:/note= "CETP intron B"

(xi) SEQUENCE DESCRIPTION: SEQ ID NO: 1:

[?]TCTTTTTC TCATAGTCAT TGTATTTTGG CCTCTTTCTA TTTATGGCAA CAGAGAGAGA 60

AAGCTTATTC CTAGATATAT GTATTTAAGT AAAAATAAAT GAATTCATGG AAACATATTA 120

AGCAATTATC CAGATAACAT AAGGGATGGC AAAAATGGTG CAGATGGTGG AGGGGAGACA 180

AGTAGAAGTT GGGGTGCTCT TGTTGAATGT CTGGCTCTGA ACTCTAGAGG AGGCCGCAGG 240

GGCTGGGCAG GAAGGAGGTG AATCTCTGGG GCCAGGAAGA CCCTGCTGCC CGGAAGAGCC 300

TCATGTTCCG TGGGGGCTGG GCGGACATAC ATATACGGGC TCCAGGCTGA ACGGCTCGGG 360

CCACTTACAC ACCACTGCCT GATAACCATG CTGGCTGCCA CAGTCCTGAC CCTGGCCCTG 420

CTGGGCAATG CCCATGCCTG CTCCAAAGGC ACCTCGCACG AGGCAGGCAT CGTGTGCCGC 480

ATCACCAAGC CTGCCCTCCT GGTGTGTAAG TATCAGTGCA TCTGTCTGCC CTGCCAGGGG 540

TCTTTTCATG GACACCCACT ATGCCAGGAG CCTCCCTGGC CTGAAGCCAG CCCTGAAGCC 600

GGCTGCCACA CTAGCCCAGA GAGAGGAGTG CCCTGGGAGG GAGATGGGCT GAGTGGAGCT 660
GTCATCACCC CCTCCTGACC TCGCCTTCAA GGTCAAGTTC TTTGGTGAGA AGGTCCTAGC 720
TGCATTGCAA ACAGCCAGGT ATAGGGATTT GTGTTTGTCT GCGACCCAGA ATCACTGGGG 780
TTCGAGTTAG GGTTCAGATC TGAGCCAGGT TAGGGGGTTA ATGTCAGGGG GTAAAGATTA 840
GGAGGTTGGT GTATATTTGG TGTTGGGGGT CACTCTATGG CCAAAGTCAG GGGTTGCCAT 900
GAGCTCAGGT GACGGAGGCT CCATCACTGA CTGTTTGTGA CTTTGCCAGC TCCCCTGGCC 960
CTCTCTGGGC CTCAGTCTCT TGCTCATATA ATAAGGGTAT AGGGAGGCTA AATGATACAA 1020
TTTCTAAAAT AGAGTATCGC CAAGTTCAAA AGCCAGAATT ATAGACCCCA GGACTACAGA 1080
CAGTGTCACA GCATCGTCTG GGTGAGGCTA GGGTTAGTGT GCGGCTGGGC TCAGGGCTGC 1140
CCCATTTGCT AGGATCGTGG GGTTCCCATG TGTCAGGATC CAGAGGCTAG GGTATGATCA 1200
GGATCTCTAG CTGGGGTCAG GGTCAGAGCT CTCTGTGTCC CCTAGAATTG CCATCAACCT 1260
TAAACCCAGA GGAGGCCCAG TCCAACCCCT CAGCTTTAAG ACCTGGGAGC CTCATCTCAG 1320
AGAGGCTGAG TCATGGCCAA GGCAGTTGGG GTGGGAGCAG GGGGCTTGGT GTGGGCCTGC 1380
AGCCCTCATC CACTGCCCTC CCTCTAGTGA ACCACGAGAC TGCCAAGGTG ATCCAGACCG 1440
CCTTCCAGCG AGCCAGCTAC CCAGATATCA CGGGCGAGAA GGCCATGATG CTCCTTGGCC 1500
AAGTCAAGTA TGGGTTGCAC AAGTGAGTCG GGCCTCGGGT GTGACCTGGC TGGGGGTAGG 1560
GTGGCGGGAG GAACAGCCTG GGCTTCCCCC AGCCACAGGG AGGAAAGGCA GCAGCTGGGG 1620
GACTCAGGTC TCTCCCCTTG ATTTGGAACC AGAGCC 1656

## Description

The present invention relates to an assay for testing the genetic predisposition to respond to LDL-cholesterol lowering therapy in patients with coronary artery disease (CAD).

Patients with clinically manifest coronary artery disease (CAD) are at high risk for recurrent cardiovascular events. The prevention of such events involves a wide range of measures, including cholesterol-(or lipid-)lowering therapy. The latter has been demonstrated to significantly reduce total mortality in male patients with CAD. Despite the potential of cholesterol-lowering drugs to retard progression of coronary atherosclerosis, and reduce cardiovascular events, myocardial infarctions are found not to be prevented in a substantial percentage of these patients. The understanding why not all patients benefit from such therapy is limited. Both environmental and genetic factors are thought to contribute to this lack of response to cholesterol-lowering strategies.

It is the object of the present invention to provide a means to predict the susceptibility of a subject to cholesterol-lowering therapy.

In order to achieve this object, a large cohort of 807 males with angiographically documented coronary atherosclerosis randomized to treatment with an HMG-CoA reductase inhibitor (pravastatin) or placebo was studied. The primary objective of this study was to examine the relationship between a frequent variant of the CETP gene (TaqIB), the progression of coronary atherosclerosis, and its influence on cholesterol-lowering therapy.

The cholesterol ester transfer protein (CETP), plays a key role in high-density lipoprotein (HDL) metabolism. It mediates the exchange of lipids between lipoproteins resulting in net transfer of cholesteryl ester from HDL to other lipoproteins and in subsequent uptake of cholesterol by hepatocytes. This flux of cholesterol towards the liver is known as reverse cholesterol transport. By increasing the cholesteryl ester content of low-density and very-low density lipoproteins, CETP promotes the atherogenicity of these lipoproteins. In addition, high plasma levels of CETP associated with reduced concentrations of HDL cholesterol are a strong and independent risk factor for atherosclerotic vascular disease. These data and other recent studies in both humans and animals support the notion that CETP can contribute to atherogenesis.

Polymorphisms of the CETP gene have been used previously as a tool to investigate the intricate association between CETP, HDL cholesterol and CAD. The majority of single locus association studies, and a number of sib-pair linkage analysis studies have confirmed that heterogeneity at the CETP gene locus in part determines HDL cholesterol levels in plasma. Others have shown that this association is gender specific and that the relationship is influenced by environmental factors. However, none of the studies up to date have been involved with predicting the success of lipid-lowering therapies in patients.

One specific polymorphism in the CETP gene, mostly referred to as TaqIB, is associated with an effect on lipid transfer activity, and HDL cholesterol levels. Recently it was reported that this polymorphism is also associated with plasma CETP concentrations in healthy Caucasian males.

According to the invention, the screening for the presence (B1) or absence (B2) of a TaqI restriction site in intron 1 of the CETP gene in the DNA of all participants, revealed frequencies for the B1B1, B1B2 and B2B2 genotypes in the whole cohort of 35%, 49% and 16%, respectively, which demonstrates that this gene marker exhibits a high level of heterozygosity and is, in fact, a very common genetic variation among Caucasian subjects.

While assessing the effect of pravastatin on the development of CAD in relation to the TaqIB polymorphism, a novel interaction was revealed between lipid-lowering treatment and this CETP gene variant in predicting the progression of coronary atherosclerosis. The association of the B1-allele with progression of CAD, as observed in the placebo group, was actually reversed in pravastatin-treated individuals.

While comparing the TaqIB subgroups in both arms of the study, a dose-dependent relationship was observed between the B1-allele and the ability of pravastatin to induce regression of coronary atherosclerosis. In contrast to findings in the placebo-treated group, carriers of the B1B1, B1B2 and B2B2 genotypes in the pravastatin group presented lowest, intermediate and highest progression of CAD. In other words, the response to pravastatin with regard to coronary atherosclerosis was greatest for homozygotes for B1B1 while B2B2 homozygotes did not appear to benefit from this treatment.

This genetic predisposition to the response to LDL-lowering drugs constitutes a novel and highly important observation of this study. According to the invention, the TaqIB polymorphism of CETP is found to be a genetic marker for identifying subjects who will or will not exhibit regression of coronary atherosclerosis in response to LDL cholesterol-lowering therapy.

The present invention is based on this finding and provides an assay for testing the genetic predisposition to respond to lipid-lowering therapy in patients with coronary artery disease (CAD), comprising:
a) identifying the presence or absence of a TaqIB restriction site in intron 1 in both alleles of the cholesteryl ester transfer protein (CETP) gene by suitable molecular biological techniques;
b) correlating the presence of the restriction site on one or both alleles with a high or intermediate, susceptibility for lipid-lowering therapy.

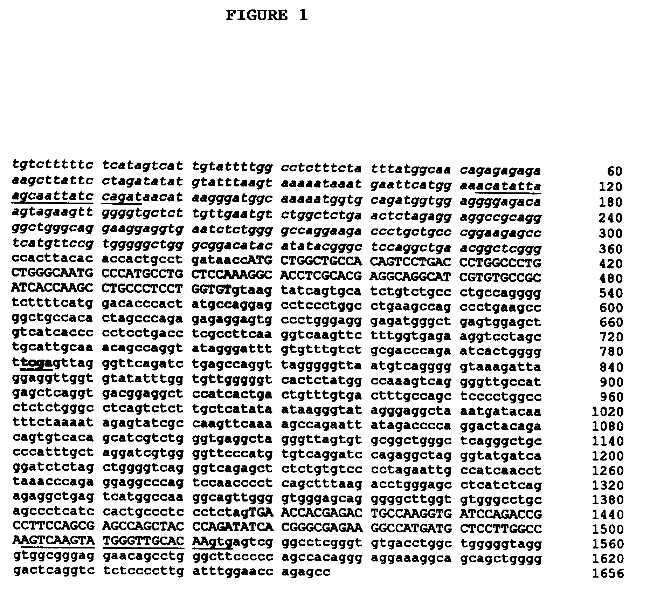

Preferably, the suitable molecular biological technique is the Polymerase Chain Reaction (PCR) technique using the following primers:
Forward: 5'-ACA TAT TAA GCA ATT ATC CAG AT-3'
Reverse : 5'-CAC TTG TGC AAC CCA TAC TTG ACT-3'
The genomic region that is relevant for the present invention is located around the TaqI restriction site in intron 1 of the CETP gene. The above identified primers are located such that after digestion of the amplified fragment two DNA fragments are obtained that can be found on an agarose gel after electrophoresis. However based on the genomic sequence given in figure 1, which represents part of the CETP gene, comprising intron 1 in which the TaqI restriction site, if present, is located, suitable other primers can very well be designed by the average skilled person. The genomic fragment depicted in figure 1 is sequenced by Agellon et al. (Biochemistry 29, 1372-1376 (1990)) and deposited in the GenBank/EMBL database (Intelligenetics, Mountain View, CA, and European Molecular Biology Laboratories, Heidelberg, Germany under accession number J02898. The general structure of the genomic fragment is as follows:

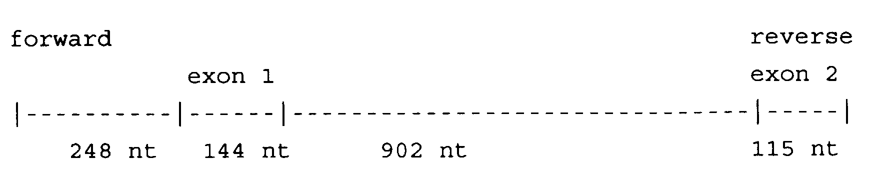
forward                                    reverse
                exon 1                      exon 2
     248 nt    144 nt        902 nt         115 nt

The invention further relates to a testkit for performing the assay, comprising a set of PCR primers derived from the CETP gene, an amplification buffer and thermostable DNA polymerase. Preferably the PCR primers are the following:
Forward: 5'-ACA TAT TAA GCA ATT ATC GAG AT-3'
Reverse : 5'-CAC TTG TGC AAC CCA TAC TTG ACT-3'
and are to be used in a final concentration of 0.5 µmol/l, and the amplification buffer consists of 10 mmol/l Tris-HCl (pH 9.0), 50 mmol/l KCl, 0.1% wt/vol gelatin, 1.5 mmol/l MgCl₂, 1% Triton X-100, 100 to 200 µmol/l, 20 mg/dl bovine serum albumin. The kit further comprises thermostable DNA polymerase to be used in a concentration of 0.3 to 0.5 U.

The invention also relates to a genetic marker that can predict the angiographic response to lipid-lowering therapy. This genetic marker is the B1-allele of the CETP gene, which gene marker has essentially the nucleotide sequence of the genomic DNA fragment as depicted in figure 1, in which sequence nucleotides 782 to 785 represent a TaqI restriction site (TCGA) or not. In the previous case the individual tested will probably be susceptible to lipid lowering therapies. In the latter case the individual may not be susceptible. In the latter case, the gene marker has essentially the nucleotide sequence of the genomic fragment as depicted in figure 1, in which sequence nucleotides 782 to 785 differ from a TaqI restriction site (TCGA).

Since quite recent international consensus has been reached regarding the treatment of patients with established coronary artery disease. The National Cholesterol Education Programme of the USA and the European Atherosclerosis Society Guidelines now advocate prescription of cholesterol lowering drugs (HMG CoA reductase inhibitors) to essentially all patients with CAD for secondary prevention. In the present study, however, 16% of these patients carry a genetic marker that predicts no response to such therapy. These results have two major consequences.

Firstly, when 16% of these patients would have been omitted from recent cholesterol lowering megatrials (Four-S, CARE, LIPID and WOSCOPS) the results would have dramatically improved and shown even greater reductions in CAD mortality and morbidity. Therefore, the use of the CETP gene marker becomes important for both patients and the pharmaceutical industry in the future design of trials. The invention also relates to this type of use of the marker.

Secondly, governments try to restrict the use of lipid-lowering drugs in patients for whom cost-effectiveness is questionable. Use of the CETP gene marker can effectively identify a subset of patients for whom the use of HMG CoA reductase inhibitors is not beneficial. The invention thus also relates to this use of the genetic marker.

The present invention will be further illustrated in the following examples. Example 1 presents the study on which the invention is based. The other example demonstrates a practical embodiment of the assay of the invention.

In the Examples reference is made to the accompanying figures in which:
figure 1 shows the nucleotide sequence of the genomic DNA fragment encompassing intron 1 of the cholesterol ester transfer protein (CETP). Exons are printed in upper case, introns in lower case. The 5' non-coding region is printed in italic. The primary transcript starts at position 361. The regions to which the PCR primers are directed are underlined, and the location of the Taq1 restriction site (782-785) is underlined and printed in bold face.

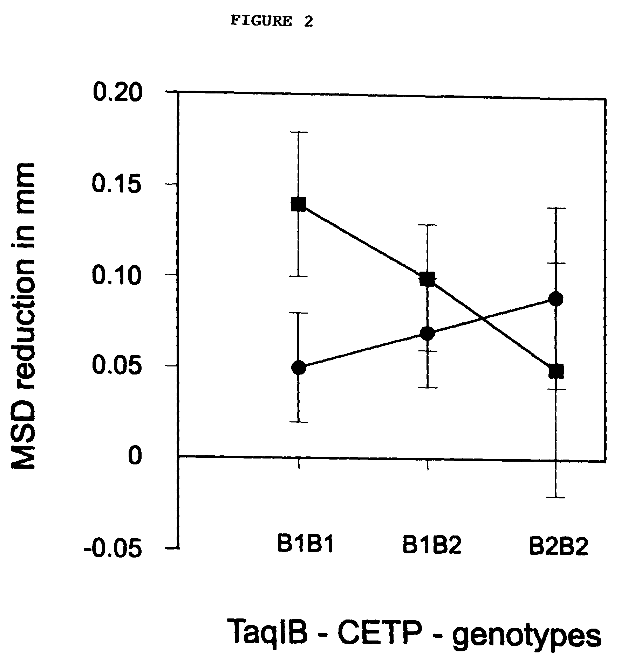

figure 2 shows Mean Segment Diameter Reduction (MSDR) according to the TaqIB genotype in patients with established coronary atherosclerosis treated with either placebo or pravastatin. The squares represent the mean segment diameter values for the placebo-treated patients, whereas the circles give these values for the patients treated with pravastatin. For each value this illustration gives the 95% confidence intervals as solid lines for the placebo group and as dotted lines for the pravastatin group, respectively. Abbreviations: B1 = presence of TaqIB restriction site in intron 1 of the CETP gene; B2 = absence of TaqIB restriction site; MSD, mean segment diameter reduction, reflecting diffuse development of coronary atherosclerosis.

### EXAMPLES

### EXAMPLE 1

### Correlation between allele B1 and susceptibility to cholesterol-lowering therapy

### 1. Introduction

The DNA of 807 male patients with angiographically documented coronary atherosclerosis was analyzed for the presence or absence of a polymorphism in the gene coding for CETP. This DNA variation was denoted as B1 and B2, respectively. All patients participated in a cholesterol-lowering coronary regression trial and were randomized to treatment with either pravastatin or placebo for a period of two years.

It was then found that the B1-variant of the CETP gene was associated with both higher plasma CETP and lower HDL cholesterol concentrations. In addition, a significant dose-dependent association between this marker and the progression of coronary atherosclerosis became evident in the placebo group. This association was neutralized by pravastatin, and while this drug significantly reduced low-density lipoprotein cholesterol and CETP levels, 16 percent of the patients on pravastatin, i.e. homozygotes for B2B2, did not exhibit retardation of progression of coronary atherosclerosis.

### 2. Materials and methods

### 2.1 Subjects with CAD

807 Male patients participating in the Regression Growth Evaluation Statin Study (REGRESS), which has previously been described in detail (Kuivenhoven et al., Arterioscler. Thromb. Vasc. Biol. 17:560-568 (1997)) were prospectively studied. In short, study participants were required to have at least one coronary artery with a stenosis off >50 percent as assessed by coronary angiography, a plasma total cholesterol concentration of 155-310 mg per deciliter (4 to 8 millimole per liter) and triglycerides of 350 mg per deciliter(< 4 millimole per liter). Patients were subsequently randomized to treatment with pravastatin sodium (Pravachol®; 40 mg/day ) or to placebo for a period of two years. The placebo group contained 396 patients and the group treated with pravastatin contained 411 patients.

Computer-assisted quantitative coronary angiography was carried out at the start and at the end of the study. The two following primary measures of outcome were used: (a) the change in the average Mean Segment Diameter (MSD) per patient (also known as Mean Lumen Diameter) reflecting diffuse changes of atherosclerosis, and (b) the change in the average Minimum Obstruction Diameter (MOD) Minimum Lumen Diameter) per patient (also known as Minimum Lumen Diameter) which reflects the focal change of atherosclerosis. Given these definitions, one should note that a greater decrease in MSD and/or MOD reflects more progression of coronary atherosclerosis.

### 2.2 DNA analysis

Blood was collected at baseline and DNA extracted following standard procedures (Sambrook et al. (1989) Molecular Cloning, A Laboratory Manual, 2^{nd} Ed., Cold Spring Harbor Laboratory, Cold Spring Harbor, NY). The polymerase chain reaction-based method to screen for the TaqIB polymorphism in intron 1 of the CETP gene was carried out as described previously (Kuivenhoven et al., supra).

### 2.3 Biochemical analyses

Plasma lipids and lipoproteins were measured using standard techniques (Groenemeyer et al., Circulation 95:2628-2653 (1997)). Due to a lack of sufficient aliquots of plasma, CETP concentrations were determined in 237 patients at baseline, and in only 68 pravastatin-treated subjects at the end of the trial. These measurements were performed by solid phase radioimmunoassay using recombinant human protein (provided by Dr. A.Tall; Columbia University, New York) as standard and a CETP monoclonal antibody, TP2 (produced by Dr. R. Milne; University of Ottawa Heart Institute; Ottawa, McPherson et al., Arterioscler. Thromb 11:797-804 (1991). In addition, the lipase and lipoprotein lipase activities were measured as reported (Groenemeyer et al., supra).

### 2.4 Statistical Analysis

Differences in baseline clinical characteristics, lipids, lipoproteins, lipolytic enzymes and plasma CETP concentrations among the TaqIB subgroups were quantified with means and standard deviations, or with numbers and percentages.

Since triglyceride levels had a skewed distribution, the statistical analyses were based on log transformed data. However, the triglycerides levels in Tables 2 and 3 are given as means and standard deviations since these data are easy to interpret, and the observed means were very similar to the median values. The differences between carriers of the three TaqIB genotypes (i.e. B1B1, B1B2, and B2B2) were tested using one-way ANOVA or Pearson's Chi-square test. Changes in lipids, lipoproteins and angiographic parameters during the trial period were quantified with means and standard deviations, and the differences within the two medication groups were tested using one-way covariance analysis with baseline levels as covariates.

The interaction between the three CETP TaqIB genotypes and medication (placebo/pravastatin) was tested using the interaction test of two-way covariance analysis. It was checked whether the above interaction between genotype and medication was dependent of baseline HDL cholesterol concentration, changes in HDL during the trial, baseline MSD, baseline MOD, and each of hepatic lipase or lipoprotein lipase activity by using these as covariates in two-way covariance analysis. The latter analysis was also carried out to test if the significant differences in decrease in either MSD or MOD as identified amongst the TaqIB subgroups in the placebo group, were dependent on the above parameters.

Differences in rate of events between genotypes were analyzed with Pearson Chi-square tests within the medication groups, and the interaction between genotype and medication with logistic regression. Within each of the patient groups, the Hardy-Weinberg equilibrium was tested by means of gene counting and χ² analysis. Throughout, a P value of 0.05 was interpreted as representing a statistically significant difference. All statistical analyses were carried out with the Statistical Analysis System (SAS, version 6.1, SAS Institute, Cari, NC) and adapted from the EGRET Manual, (Statistical and Epidemiological Research Corporation, 1991, Seattle, Washington, USA).

### 3. Results

### 3.1 Frequency of the TaqIB CETP polymorphism

B1 and B2 were used to denote the presence and absence of a restriction site for the enzyme TaqI in intron 1, respectively. In the total cohort, the B1 and B2 alleles were found at frequencies of 0.594 and 0.406, respectively. These frequencies did not differ significantly in both arms of the study (data not shown). For each of the placebo group, the pravastatin group as well as for the total cohort, the observed frequencies were in Hardy-Weinberg equilibrium.

### 3.2 Baseline characteristics in patients with different TaqIB genotypes

Table 1 illustrates the absence of statistically significant differences between patients of either TaqIB genotype at baseline with respect to risk factors for CAD, severity of coronary atherosclerosis, or treatment of the disease. In each of the three groups, approximately 50 percent of the patients were randomised to cholesterol-lowering therapy with pravastatin.

The upper panel of Table 2 shows a clear association of the B1 allele with lower HDL cholesterol levels. The observed differences in plasma HDL cholesterol concentrations among the TaqIB subgroups were highly significant (P<0.0001). No differences amongst the three subgroups were found for lipoprotein lipase or hepatic lipase activities (Table 2) or other CAD risk factors including lipoprotein(a) levels, fibrinogen, and serum glucose (data not shown).

### 3.3 TaqIB and CETP concentrations in plasma

The lower panel of Table 2 illustrates that, at baseline, the B1 allele was strongly associated with higher CETP concentrations in all patients (P<0.0001). At the end of the trial, the patients randomized to pravastatin demonstrated a 16 percent reduction in plasma CETP concentrations when compared with baseline CETP concentrations (2.03±0.53 vs. 1.71±0.46 µg per milliliter; P<0.001; data not shown) . There were too few patients divided over the different TaqIB genotypes to allow meaningful conclusions about differences in reductions of CETP concentration as a result of the use of pravastatin.

### 3.4 In trial changes in lipids and lipoproteins

The upper panel of Table 3 shows the absence of an effect of the TaqIB polymorphism on the changes in total cholesterol, low-density lipoprotein, cholesterol, triglycerides, and HDL cholesterol levels in both arms of the study. Pravastatin reduced total cholesterol, low-density lipoprotein cholesterol, triglycerides and increased HDL cholesterol to a similar extent in all three subgroups of patients.

### 3.5 Angiographic disease parameters

The lower panel of Table 3 shows the angiographic disease parameters and events according to the TaqIB-CETP genotype in the placebo and pravastatin treated patients. A larger figure for either a decrease in mean segment diameter (MSD) or minimum obstruction diameter (MOD) indicates more progression of atherosclerosis.

In the placebo group patients, statistically significant differences were observed amongst the TaqIB subgroups for decreases of both MSD (P<0.03) and MOD (P<0.05). An association between the B1-allele and the degree of coronary atherosclerosis becomes apparent and a gene dosage effect is demonstrated by the most pronounced progression of atherosclerosis in the B1B1 carriers as reflected by the greatest decrease in both MSD and MOD, with B1B2 carriers exhibiting an intermediate phenotype, and B2B2 homozygotes showing least progression of atherosclerosis (i.e. lowest decreases in MOD and MSD).

In the pravastatin treated patients, the differences in decreases of MSD and MOD amongst the TaqIB subgroups did not reach statistical significance (P<0.36, P<0.38, respectively) . However, B1B1, B1B2, and B2B2 carriers showed lowest, intermediate, and highest progression in diffuse atherosclerosis (MSD). In addition, less focal atherosclerosis was observed in the B1B1 carriers as compared with carriers of both other genotypes in this group as demonstrated by the smallest reduction in MOD.

### 3.6 Comparison of placebo and pravastatin groups.

While comparing the placebo group with the pravastatin group, a significant interaction (last column Table 3) was identified between pravastatin treatment and decreases in MSD (P<0.014) and MOD (P<0.05). The interaction between the use of pravastatin and MSD is shown in figure 2. From this illustration, it is clear that the association of the B1-allele with more progression of diffuse atherosclerosis (i.e. higher reductions in MSD) as observed in the placebo group, was influenced by the use of pravastatin. In fact, the B1-allele appeared to be associated with less progression in the patients on pravastatin.

It is evident that there exists a positive dose-dependent relationship between the B1-allele and the efficacy of pravastatin to retard progression of coronary atherosclerosis. Carriers of two B1 alleles benefited most from treatment with pravastatin: they demonstrated significantly less progression of coronary atherosclerosis, that is smaller decreases of both MSD and MOD (P<0.001, P<0.002, respectively), as compared with their B1B1 counterparts in the placebo group. Furthermore, carriers of only one B1 allele (B1B2) on pravastatin only showed significantly less focal atherosclerosis (MOD; P<0.01) when compared with carriers of this genotype in the placebo group. Finally, B2B2 homozygotes showed a statistically non-significant larger progression at the end of the study as compared with their counterparts in the placebo group.

Both the association of the CETP TaqIB genotype with either the decrease in MSD or MOD in the placebo group, and the interaction between the TaqIB genotype and pravastatin treatment remained significant after adjustments were made for baseline MSD (or baseline MOD, respectively), baseline HDL cholesterol, changes in HDL cholesterol levels, and activities of both hepatic lipase and lipoprotein lipase (data not shown).

### 4. Discussion

The TaqIB polymorphism of the CETP gene was investigated on the outcome measures of a large cohort of men participating in an angiographic coronary regression study. The results indicate that this genetic marker is not only associated with the progression of coronary atherosclerosis in a dose-dependent manner but most importantly can predict the angiographic response to cholesterol-lowering therapy.

Thus, the study revealed a dose-dependent relationship between this gene variant and the progression of coronary atherosclerosis. Importantly, this relationship proved independent of HDL cholesterol levels nor was it influenced by plasma lipase activities.

In patients randomized to placebo treatment, angiographic outcome parameters demonstrated that the B1-allele is associated with increased progression of coronary atherosclerosis. Subsequently, a novel dose-dependent interaction was identified between this gene variant and the ability of pravastatin to inhibit progression of coronary atherosclerosis.

The association between the B1-allele and the progression of coronary atherosclerosis as observed in the placebo group, was not observed in the pravastatin-treated individuals. In contrast to the findings in the placebo-treated group, carriers of the B1B1, B1B2, and B2B2 genotypes in the pravastatin group presented with lowest, intermediate and highest progression of coronary atherosclerosis. In other words, the response of these patients to pravastatin with regard to coronary atherosclerosis was greatest for B1B1 homozygotes, while B2B2 homozygotes did not appear to benefit from this treatment. Thus, this study has revealed a genetic predisposition to the response to cholesterol-lowering drugs.

### EXAMPLE 2

### Assay according to the invention

Amplification of 0.1 to 0.5 µg genomic DNA is carried out in amplification buffer (10 mmol/l Tris-HCl (pH 9.0), 50 mmol/l KCl, 0.1% wt/vol gelatin, 1.5 mmol/l MgCl₂, 1% Triton X-100, 100 to 200 µmol/l, 20 mg/dl bovine serum albumin) with PCR primers (forward: 5'-ACA TAT TAA GCA ATT ATC CAG AT-3' and reverse: 5'-CAC TTG TGC AAC CCA TAC TTG ACT-3') in a final concentration of 0.5 µmol/l. The reaction volume is 50 µl. After an initial denaturation step (10 minutes, 95°C), 0.3 to 0.5 U thermostable DNA polymerase is added, followed by 30 amplification cycles of 1 minute at 95°C, 1 minute at 60°C and 1 to 2 minutes at 72°C. The reaction ends with an extension step of 10 minutes at 72°C.

10 µl of the PCR reaction is subjected to digestion with the restriction endonuclease TaqI. Next, the DNA fragments are seperated by agarose gel electrophoresis in the presence of ethidium bromide. DNA is visualized on a UV transilluminator. The presence of two DNA fragments indicates that the TaqI restriction site is present in intron 1 of the CETP gene. If this restriction site is absent in both alleles, only one DNA fragment (1413 nt) will be visible.

According to the invention the novel observation was made that the TaqIB polymorphism of the CETP gene is associated with the progression of coronary atherosclerosis. This relationship was dose-dependent and independent of HDL cholesterol levels, and plasma lipase activities. Of special importance is the finding that this marker also predicts the angiographic response to pravastatin and therefore enables the identification of subjects who will and who will not benefit from cholesterol-lowering therapy. The relevance of this finding is emphasized by the high frequency of this polymorphism; 16 percent of this male population was homozygous for the B2B2 genotype. This genetic variant of the CETP gene is therefore an important factor in designing better treatment regimens and in improving cost-effectiveness of treatment for patients with CAD.

**Table 1**

| **CETP - TaqIB - genotypes** | | | | |
|---|---|---|---|---|
| | **B1B1** (n=281) | **B1B2** (n=397) | **B2B2** (n= 129) | P* |
| | | | | |

| **Demographic characteristics** | | | | |
|---|---|---|---|---|
| Age (years): mean (SD) | 55 (8) | 57 (8) | 56 (7) | 0.21 |
| Body mass index (kg.m⁻²): mean (SD) | 26 (3) | 26 (3) | 26 (3) | 0.96 |
| Systolic bloodpressure (mmHg): mean (SD) | 135 (19) | 135 (18) | 135 (17) | 0.97 |
| Diastolic bloodpressure (mmHg): mean (SD) | 82 (10) | 82 (10) | 81 (9) | 0.58 |
| Smoking ever (yes/no): n (%) | 246 (88%) | 346 (87%) | 118 (92%) | 0.43 |
| Current smokers: n (%) | 76 (27%) | 113 (29%) | 30 (23%) | 0.50 |

| **Coronary artery disease characteristics** | | | | |
|---|---|---|---|---|
| History of myocard. infarct. (yes/no): n (%) | 129 (46%) | 183 (46%) | 68 (53%) | 0.38 |
| LV Ejection fraction (%): mean (SD)7 | 0 (13) | 71 (12) | 70 (12) | 0.99 |
| MSD (mm): mean (SD) | 2.80 (0.45) | 2.81 (0.49) | 2.81 (0.45) | 0.99 |
| NOD (mm): mean (SD) | 1.90 (0.53) | 1.92 (0.55) | 1.86 (0.56) | 0.56 |
| % Stenosis (%): mean (SD) | 35 (13) | 35 (12) | 37 (15) | 0.23 |

| Coronary artery disease: n (%) | | | | |
|---|---|---|---|---|
| 1 vessel | 115 (41%) | 165 (42%) | 54 (42%) | |
| 2 vessels | 103 (37%) | 128 (33%) | 45 (35%) | 0.77 |
| 3 vessels | 62 (22%) | 101 (26%) | 30 (23%) | |
| Pravastatin (yes/no): n (%) | 136 (48%) | 212 (54%) | 63 (49%) | 0.36 |
| Long-acting nitrates (yes/no): n (%) | 149 (53%) | 229 (58%) | 70 (54%) | 0.44 |
| β-blocking agents (yes/no): n (%) | 208 (74%) | 295 (75%) | 93 (72%) | 0.86 |
| Calcium blocking agents (yes/no): n (%) | 163 (58%) | 252 (64%) | 73 (57%) | 0.20 |

**Table 2**

| **CETP - TaqIB - genotype** | | | | |
|---|---|---|---|---|
| | **B1B1** (n=281) | **B1B2** (n=397) | **B2B2** (n=129) | *P |
| | | | | |

| **Lipids** | | | | |
|---|---|---|---|---|
| Total cholesterol (mg.dL⁻¹): mean (SD) | 234 (33) | 232 (34) | 237 (32) | 0.35 |
| HDL cholesterol (mg.dL⁻¹): mean (SD) | 34 (8) | 36 (8) | 39 (10) | <0.0001 |
| LDL cholesterol (mg.dL⁻¹): mean (SD) | 167 (31) | 166 (31) | 169 (29) | 0.66 |
| Triglycerides (mg.dL⁻¹): mean (SD) ^{H} | 151 (38) | 143 (38) | 117 (40) | 0.04 |

| **Lipase activities** | | | | |
|---|---|---|---|---|
| Lipoprotein lipase act. (U.L⁻¹): mean (SD) | 108 (45) | 112 (47) | 109 (41) | 0.63 |
| Hepatic lipase activity (U.L⁻¹): mean (SD) | 388 (114) | 381 (119) | 393 (135) | 0.65 |

| | **B1B1** (n=85) | **B1B2** (n=89) | **B2B2** (n=63) | |
|---|---|---|---|---|
| **CETP concentration** (µg.mL⁻¹⁾: mean (SD) | 2.29 (0.62) | 2.01 (0.51) | 1.76 (0.51) | <0.0001 |

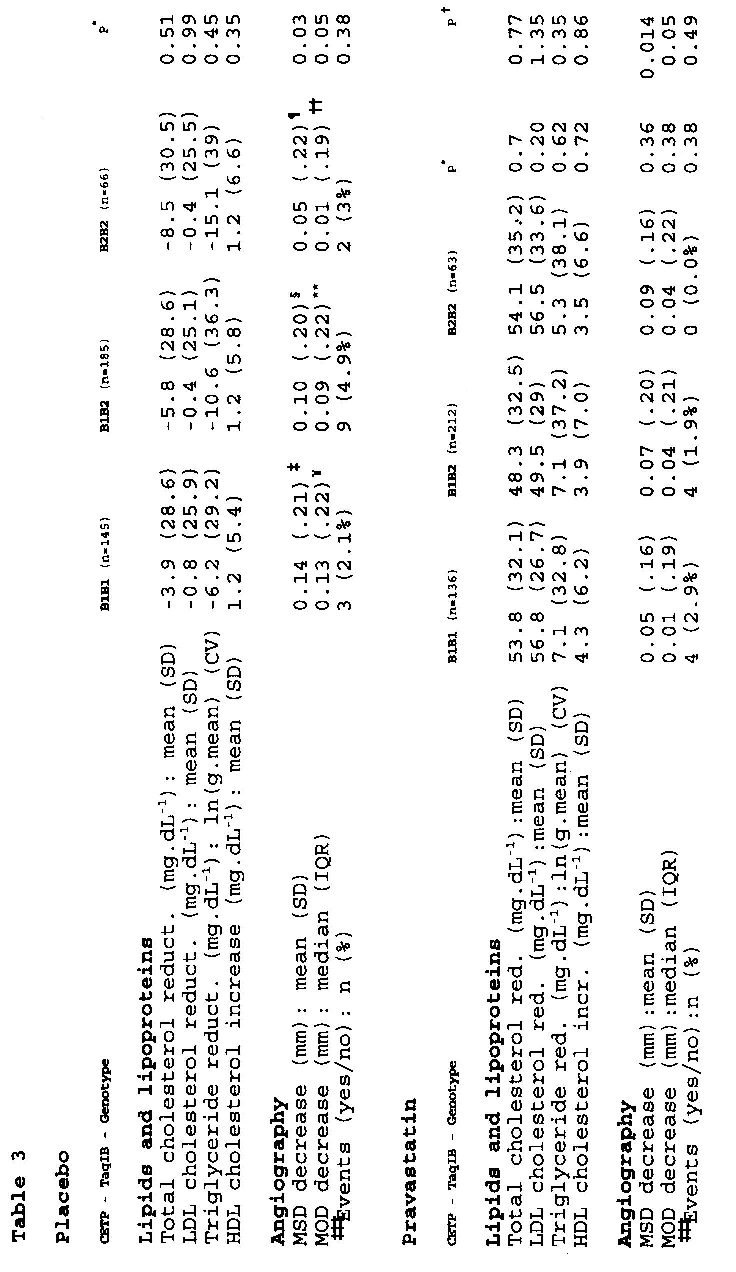

### LEGENDS TO TABLES

### TABLE 1

Abbreviations: B1 = presence of TaqIB restriction site in intron 1 of the CETP gene; B2 = absence of TaqIB restriction site; SD = standard deviation; LV = left ventricular; MSD = mean segment diameter; MOD = minimum obstruction diameter. * P value of oneway analysis of variance, or chi-square test, where appropriate, comparing the three CETP-TaqIB genotypes.

### TABLE 2

Abbreviations: B1 = presence of TaqIB restriction site in intron 1 of the CETP gene; B2 = absence of TaqIB restriction site; SD = standard deviation; * P value of oneway analysis of variance comparing the three CETP-TaqIB genotypes. ^{H} Triglycerides are given in absolute numbers, however, the natural logarithm of geometric mean was used in the statistical analysis. For conversion from mg.dL⁻¹ to mmol.L⁻¹ for total cholesterol, LDL cholesterol and HDL cholesterol levels, multiply by factor 0.02586. For conversion of triglyceride levels from mg.dL⁻¹ to mmol.L⁻¹, use 0.01129.

### TABLE 3

Abbreviations: MSD = mean segment Diameter; MOD = minimum obstruction diameter (Note: A larger figure for either a decrease in mean segment diameter (MSD) or minimum obstruction diameter (MOD) indicates more progression of atherosclerosis). SD = standard deviation; IQR = interquartile range; * P value of covariance analysis of the change measures with baseline values as covariate, or chi-square test (where appropriate), comparing the three CETP-TaqIB genotypes; ^{†} P value of the interaction test of the interaction between treatment (placebo/pravastatin) and the CETP TaqIB genotype (B1B1-B1B2-B2B2) of covariance analysis with baseline values as covariate or of logistic regression, where appropriate. P values while comparing TaqIB subgroup of the placebo group with the pravastatin group: ^{‡} P<0.001; ^{§} P<0.38; ^{¶} P<0.30; ^{¥} P<0.002; ** P<0.01; ^{†† P}<0.48. 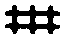 Events are defined as death and myocardial infarction. For conversion from mg.dL⁻¹ to mmol.L^{-¹} for total cholesterol, LDL cholesterol and HDL cholesterol levels, multiply by factor 0.02586. For conversion of triglyceride levels from mg.dL⁻¹ to mmol.L⁻¹, use 0.01129.

## Claims

1. Assay for testing the genetic predisposition to respond to lipid-lowering therapy in patients with coronary artery disease (CAD), comprising:
a) identifying the presence or absence of a TaqIB restriction site in intron 1 in both alleles of the cholesteryl ester transfer protein (CETP) gene by suitable molecular biological techniques;
b) correlating the presence of the restriction site on one or both alleles with a high or intermediate, susceptibility for lipid-lowering therapy.

2. Assay as claimed in claim 1, wherein the suitable molecular biological technique is the following:
a) amplifying the gene in a polymerase chain reaction using the following primers:
Forward: 5'-ACA TAT TAA GCA ATT ATC CAG AT-3'
Reverse : 5'-CAC TTG TGC AAC CCA TAC TTG ACT-3'
to obtain a PCR reaction product;
b) digesting the PCR reaction product with the restriction endonuclease TaqI in a reaction mixture;
c) performing agarose gel electrophoresis with the reaction mixture in the presence of a means to visualize the complete PCR reaction product and/or digested DNA fragments thereof; and
d) visualizing the PCR reaction product and/or digested fragments thereof;
and the presence of the restriction site on one or both alleles is established by finding two DNA fragments.

3. Testkit for performing the assay as claimed in claim 1 or 2, comprising a set of PCR primers derived from the CETP gene, an amplification buffer and thermostable DNA polymerase.

4. Testkit as claimed in claim 3, wherein the set of PCR primers is derived from the CETP genomic fragment depicted in figure 1.

5. Testkit as claimed in claim 3 or 4, wherein the PCR primers are the following:
Forward: 5'-ACA TAT TAA GCA ATT ATC CAG AT-3'
Reverse : 5'-CAC TTG TGC AAC CCA TAC TTG ACT-3'
and are to be used in a final concentration of 0.5 µmol/l, and the amplification buffer consisting of 10 mmol/l Tris-HCl (pH 9.0), 50 mmol/l KCl, 0.1% wt/vol gelatin, 1.5 mmol/l MgCl₂, 1% Triton X-100, 100 to 200 µmol/l, 20 mg/dl bovine serum albumin, and 0.3 to 0.5 U thermostable DNA polymerase.

6. Cholesterol ester transfer protein (CETP) gene marker for identifying individuals being susceptible for lipid-lowering therapies, which gene marker has essentially the nucleotide sequence of the genomic DNA fragment as depicted in figure 1, in which sequence nucleotides 782 to 785 represent a TaqI restriction site (TCGA).

7. Cholesterol ester transfer protein (CETP) gene marker for identifying individuals that may not be susceptible for lipid-lowering therapies, which gene marker has essentially the nucleotide sequence of the genomic DNA fragment as depicted in figure 1, in which sequence nucleotides 782 to 785 differ from a TaqI restriction site (TCGA).
